# EUROPEAN PATENT APPLICATION

(11) **EP 2 710 997 A1**
(43) Date of publication of application: **26.03.2014**
(21) Application number: 12786175.5
(22) Date of filing: 15.05.2012
(51) Int. Cl.: A61K 8/34, A61K 8/37, A61Q 1/14

(54) **OIL COMPOSITION FOR COSMETICS MATERIAL**

(30) Priority: 18.05.2011 JP 2011110958
(71) Applicant: Daicel Corporation, Osaka 530-0001 (JP)
(72) Inventor: MAEHARA, Tetsuya, Ohtake-shi, Hiroshima 739-0695 (JP)
(74) Representative: Grünecker, Kinkeldey, Stockmair & Schwanhäusser
(86) International application number: PCT/JP2012/062371
(87) International publication number: WO 2012/157628

(57) **Abstract**

An oil composition for cosmetics material which can, either on wet or dry skin, readily dissolve makeup dirt out and be freshly rinsed off by washing with water without leaving an oily feel, and which has excellent usability, safety, and stability over time is provided. The oil composition for cosmetics material of the present invention comprises 0.1 to 4.3% by weight of glycerol fatty acid ester, 0.5 to 25.7% by weight of polyglycerol fatty acid ester, 1 to 30% by weight of polyglycerol monoalkyl ether, and 40 to 98.4% by weight of at least one oily component selected from silicone oil, ester oil, liquid oil, hydrocarbon, and fatty acid.

## Description

### Technical Field

The present invention relates to an oil composition for cosmetics material which comprises a surfactant and an oily component capable of forming fine emulsified particles via a lamellar liquid crystalline phase in the phase transition, and which is particularly useful as a cleansing agent for oily cosmetics.

### Background Art

In the field of cosmetics, makeup removing-cleansing cosmetics are available in various types from those comprising large amounts of oil agents, such as cream, emulsion, oil and oily gel types, to those comprising none or trace amounts of oil agents, such as lotion and aqueous gel types.

A type of cleansing cosmetics comprising large amounts of oil agents can be readily miscible with oily dirt components on the skin surface and dissolve them out when used on dry skin, thereby capable of exerting excellent detergency; however, such a type of cleansing cosmetics leaves a greasy feel even after washing with water due to residual oily components, thus requiring another cleansing with a face wash and the like. Also, when such a type of cleansing cosmetics is used on wet skin, it becomes less miscible with oily dirt components on the skin surface, resulting in drastically reduced detergency. Therefore, there was a problem that such a type of cleansing cosmetics was particularly not suitable for use in a bath. Meanwhile, a problem of a type of cleansing cosmetics comprising none or trace amounts of oil agents was that although it left a reduced greasy feel (oily feel) after washing with water, it had weak detergency.

In order to solve these problems, an aqueous gel cleansing agent comprising polyoxyethylene fatty acid ester as a surfactant has been reported (see Patent Literatures 1 and 2). However, sufficient detergency has not yet been achieved, and not only that, there was a problem from a safety standpoint that polyoxyethylene fatty acid ester was highly irritating. Moreover, there was another problem of poor storage stability due to the inclusion of fatty acid in the molecule.

As a method for addressing safety, Patent Literature 3 describes a cleansing cosmetic comprising, as a surfactant, a specific polyglycerol fatty acid ester that can form a lamellar liquid crystalline phase. However, there was a problem that the aforementioned specific polyglycerol fatty acid ester had poor stability over time due to its susceptibility to hydrolysis upon addition of water.

As a method for addressing problems of impaired detergency and poor feeling upon application on wet skin, which are weaknesses associated with oily cleansing cosmetics, a method of using, as a surfactant, a specific polyglycerol fatty acid ester capable of forming a bicontinuous microemulsion, in which the oil and aqueous phases are both in a continuous state, has recently been developed (Patent Literatures 4 and 5). However, because the aforementioned specific polyglycerol fatty acid ester had low surfactant capacity for its being a complex mixture comprising polyglycerol derivative without a hydrophobic group or unintended polysubstituted bodies, it needed to be added in a large amount in order to achieve sufficient detergency. As a result, these products were highly irritating to the skin with a risk of causing inflammation. Also, owing to low viscosity, these products had a problem of dripping down when poured onto the palm of the hand, and further, achieving a gel-like viscosity in these products was also difficult.

### Citation List

### Patent Literature

Patent Literature 1: Japanese Patent Laid-Open No. H04-5213
Patent Literature 2: Japanese Patent Laid-Open No. H08-143420
Patent Literature 3: Japanese Patent Laid-Open No. 2007-23025
Patent Literature 4: Japanese Patent Laid-Open No. 2005-162691
Patent Literature 5: Japanese Patent Laid-Open No. 2010-280644

### Summary of Invention

### Technical Problem

Accordingly, an object of the present invention is to provide an oil composition for cosmetics material which can, either on wet or dry skin, readily dissolve makeup dirt out and be freshly rinsed off by washing with water without leaving an oily feel, and which has excellent usability, safety, and stability over time, and another object of the present invention is to provide a cleansing cosmetic comprising the oil composition for cosmetics material.

### Solution to Problem

The present inventors conducted intensive studies to achieve the aforementioned objects, and as a result, found the followings: a surfactant obtained by mixing a specific glycerol fatty acid ester, polyglycerol fatty acid ester, and polyglycerol monoalkyl ether at a specific ratio formed fine emulsified particles via a lamellar liquid crystalline phase, which can retain a large amount of oil phase in the liquid crystal and has excellent water solubility, in the phase transition, and thus when this surfactant was used on wet skin surface, it could form a lamellar liquid crystalline structure and be readily miscible with oily dirt components such as makeup dirt to dissolve them out; when rinsed off with water, it formed fine emulsified particles incorporating the aforementioned oily dirt components and could be readily cleansed and removed from the skin; and moreover, it had excellent safety. The present invention was completed based on these findings.

That is, the present invention provides an oil composition for cosmetics material, comprising
0.1 to 4.3% by weight of glycerol fatty acid ester represented by the following formula (1) :

R₁COO-C₃H₆O₂-H (1)

wherein R₁ represents a linear or branched aliphatic hydrocarbon group having 8 to 22 carbon atoms that optionally has a hydroxyl group,
0.5 to 25.7% by weight of polyglycerol fatty acid ester represented by the following formula (2):

R₂COO-(C₃H₆O₂)n₁-H (2)

wherein R₂ represents a linear or branched aliphatic hydrocarbon group having 8 to 22 carbon atoms that optionally has a hydroxyl group, and n₁ represents an average degree of polymerization of glycerol, which is 2 to 10,
1 to 30% by weight of polyglycerol monoalkyl ether represented by the following formula (3) :

R₃O-(C₃H₆O₂)n₂-H (3)

wherein R₃ represents a linear or branched alkyl group having 8 to 22 carbon atoms that optionally has a hydroxyl group, and n₂ represents an average degree of polymerization of glycerol, which is 3 to 20, and
40 to 98.4% by weight of at least one oily component selected from silicone oil, ester oil, liquid oil, hydrocarbon, and fatty acid.

The present invention also provides a cleansing cosmetic comprising the aforementioned oil composition for cosmetics material.

### Advantageous Effects of Invention

The oil composition for cosmetics material of the present invention forms fine emulsified particles via a lamellar liquid crystalline phase in the phase transition, and therefore, even when it is used on wet skin, it can be readily miscible with oily dirt components such as makeup dirt to lift and dissolve them out. Subsequently, when rinsed off with water, the oil composition for cosmetics material according to the present invention forms fine emulsified particles enclosing the aforementioned oily dirt components in the center thereof, thereby being easily removed from the skin and freshly rinsed off without leaving an oily feel.

Because the cleansing cosmetic of the present invention comprises the aforementioned oil composition for cosmetics material, it attains excellent detergency and feeling upon application, and can also be used in a bath. Also, a cleansing cosmetic of desired type such as a cream, emulsion, oil, or oily gel type cleansing cosmetic can be produced by adjusting the content of the oily component. Thus, the cleansing cosmetic of the present invention is particularly useful as a cleansing agent for oily cosmetics.

In the present invention, a lamellar liquid crystalline phase refers to such a phase that has a structure in which a bimolecular membrane, which is formed through the self-assembling behavior of amphiphilic molecules having both a hydrophilic group and a hydrophobic group within the molecule, and water are alternatively arranged, and is in a mesomorphic state between a crystal and a fluid to retain fluidity, which is a characteristic of a fluid, while maintaining the regularity of molecular arrangement, which is a characteristic of a solid. Because a lamellar liquid crystalline phase shows such a unique optical property as optical anisotropy, a lamellar liquid crystalline phase can be confirmed by observation with a polarizing plate or polarizing microscope.

### Description of Embodiments

### [Glycerol fatty acid ester]

The glycerol fatty acid ester of the present invention is represented by the aforementioned formula (1). In the formula (1), R₁ represents a linear or branched aliphatic hydrocarbon group having 8 to 22 carbon atoms that optionally has a hydroxyl group.

In the formula (1), the structure of C₃H₆O₂ is represented by either of the following formulae (4) and (5).

-CH₂-CHOH-CH₂O- (4)

-CH (CH₂OH) CH₂O- (5)

R₁ represents a linear or branched aliphatic hydrocarbon group having 8 to 22 carbon atoms that optionally has a hydroxyl group. Examples of the linear aliphatic hydrocarbon group having 8 to 22 carbon atoms include a C₈₋₂₂ linear alkyl group such as n-octyl, n-nonyl, n-decyl, n-undecyl, n-dodecyl (lauryl), n-tridecyl, n-tetradecyl (myristyl), n-pentadecyl, n-hexadecyl, n-heptadecyl, n-stearyl, n-nonadecyl, n-eicosyl, n-heneicosyl, and n-docosyl groups; a C₈₋₂₂ linear hydroxyalkyl group, wherein one or more hydroxyl groups are added to the aforementioned C₈₋₂₂ linear alkyl group; a C₈₋₂₂ linear alkenyl group such as n-octenyl, n-nonenyl, n-decenyl, n-undecenyl, n-dodecenyl, n-tridecenyl, n-tetradecenyl, n-pentadecenyl, n-hexadecenyl, n-heptadecenyl, n-oleyl, n-nonadecenyl, n-eicocenyl, n-heneicocenyl, and n-dococenyl groups; and a C₈₋₂₂ linear hydroxyalkenyl group, wherein one or more hydroxyl groups are added to the aforementioned C₈₋₂₂ linear alkenyl group.

Examples of the branched aliphatic hydrocarbon group having 8 to 22 carbon atoms that optionally has a hydroxyl group include a C₈₋₂₂ branched alkyl group such as isooctyl, s-octyl, t-octyl, isononyl, s-nonyl, t-nonyl, isodecyl, s-decyl, t-decyl, isoundecyl, s-undecyl, t-undecyl, isododecyl, s-dodecyl, t-dodecyl, isotridecyl, s-tridecyl, t-tridecyl, isotetradecyl, s-tetradecyl, t-tetradecyl, isopentadecyl, s-pentadecyl, t-pentadecyl, 2-hexyldecyl, isohexadecyl, s-hexadecyl, t-hexadecyl, isoheptadecyl, s-heptadecyl, t-heptadecyl, isostearyl, isononadecyl, s-nonadecyl, t-nonadecyl, isoeicosyl, s-eicosyl, t-eicosyl, isoheneicosyl, s-heneicosyl, t-heneicosyl, isodocosyl, s-docosyl, and t-docosyl; a C₈₋₂₂ branched hydroxyalkyl group, wherein one or more hydroxyl groups are added to the aforementioned C₈₋₂₂ branched alkyl group; a C₈₋₂₂ branched alkenyl group such as isooctenyl, s-octenyl, t-octenyl, isononenyl, s-nonenyl, t-nonenyl, isodecenyl, s-decenyl, t-decenyl, isoundecenyl, s-undecenyl, t-undecenyl, isododecenyl, s-dodecenyl, t-dodecenyl, isotridecenyl, s-tridecenyl, t-tridecenyl, isotetradecenyl, s-tetradecenyl, t-tetradecenyl, isopentadecenyl, s-pentadecenyl, t-pentadecenyl, isohexadecenyl, s-hexadecenyl, t-hexadecenyl, isoheptadecenyl, s-heptadecenyl, t-heptadecenyl, isooleyl, isononadecenyl, s-nonadecenyl, t-nonadecenyl, isoeicocenyl, s-eicocenyl, t-eicocenyl, isoheneicocenyl, s-heneicocenyl, t-heneicocenyl, isodococenyl, s-dococenyl, and t-dococenyl; and a C₈₋₂₂ branched hydroxyalkenyl group, wherein one or more hydroxyl groups are added to the aforementioned C₈₋₂₂ branched alkenyl group.

Among those exemplified above, R₁ in the present invention is preferably a linear or branched aliphatic hydrocarbon group having 8 to 20 carbon atoms, particularly preferably a linear aliphatic hydrocarbon group having 16 to 18 carbon atoms (particularly, a linear alkenyl group and a linear alkyl group) in view of inexpensiveness and a liquid crystal formation-promoting ability.

Specific examples of the glycerol fatty acid ester in the present invention include glycerol monooleate. In the present invention, for example, commercially available products such as those available under the trade names "RIKEMAL S-100" and "RIKEMAL OL-100 (E)" (both are available from Riken Vitamin Co., Ltd.) may also be used.

### [Polyglycerol fatty acid ester]

The polyglycerol fatty acid ester of the present invention is represented by the aforementioned formula (2). In the formula (2), R₂ represents a linear or branched aliphatic hydrocarbon group having 8 to 22 carbon atoms that optionally has a hydroxyl group. The n₁ represents an average degree of polymerization of glycerol, which is 2 to 10.

In the formula (2), the structure of C₃H₆O₂ in a parenthesis is represented by either of the aforementioned formulae (4) and (5).

R₂ represents a linear or branched aliphatic hydrocarbon group having 8 to 22 carbon atoms that optionally has a hydroxyl group, and examples thereof include those exemplified in connection with R₁ above. Among those exemplified above, R₂ in the present invention is preferably a linear or branched aliphatic hydrocarbon group having 8 to 20 carbon atoms, particularly preferably a linear aliphatic hydrocarbon group having 16 to 18 carbon atoms (particularly, a linear alkenyl group and a linear alkyl group) in view of inexpensiveness and a liquid crystal formation-promoting ability.

The n₁ represents an average degree of polymerization of glycerol, which is 2 to 10, preferably 2 to 5, and particularly preferably 2. There is a tendency that when n₁ is below the aforementioned range, oil solubility becomes too high, while water solubility is reduced. Meanwhile, there is a tendency that when n₁ exceeds the aforementioned range, water solubility becomes too high, while oil solubility is reduced.

Specific examples of the polyglycerol fatty acid ester in the present invention include diglycerol monooleate. In the present invention, for example, commercially available products such as those available under the trade names "POEM DL-100" and "RIKEMAL DO-100" (both are available from Riken Vitamin Co., Ltd.) may also be used.

### [Polyglycerol monoalkyl ether]

The polyglycerol monoalkyl ether of the present invention is represented by the aforementioned formula (3). In the formula (3), R₃ represents a linear or branched alkyl group having 8 to 22 carbon atoms that optionally has a hydroxyl group. The n₂ represents an average degree of polymerization of glycerol, which is 3 to 20.

In the formula (3), the structure of C₃H₆O₂ in a parenthesis is represented by either of the aforementioned formulae (4) and (5).

R₃ represents a linear or branched alkyl group having 8 to 22 carbon atoms that optionally has a hydroxyl group, and examples thereof include those exemplified as the linear or branched alkyl groups in connection with R₁ as above. Among those exemplified above, R₃ of the present invention is preferably a linear or branched alkyl group having 8 to 20 carbon atoms, particularly preferably a branched alkyl group having 16 to 18 carbon atoms in view of inexpensiveness and a liquid crystal formation-inhibiting ability.

The n₂ represents an average degree of polymerization of glycerol, which is 3 to 20, preferably 4 to 15, and particularly preferably 5 to 10. There is a tendency that when n₂ is below the aforementioned range, oil solubility becomes too high, while water solubility is reduced. Meanwhile, there is a tendency that when n₂ exceeds the aforementioned range, water solubility becomes too high, while oil solubility is reduced.

Examples of the polyglycerol monoalkyl ether in the present invention include pentaglycerol monoisostearyl ether, hexaglycerol mono 2-hexyldecyl ether, hexaglycerol monoisostearyl ether, heptaglycerol monoisostearyl ether, decaglycerol mono 2-hexyldecyl ether, and decaglycerol monoisostearyl ether. These can be used each alone or in combination of two or more of them.

Examples of the production method of polyglycerol monoalkyl ether include, but are not particularly limited to, (1) a method including adding glycidol to aliphatic alcohol at a specific mole ratio (the aliphatic alcohol/glycidol) and carrying out reactions in the presence of a basic catalyst, (2) a method of reacting an α-olefin epoxide with polyglycerol, and (3) a ring-opening method of alkyl glycidyl ether using polyglycerol in the presence of an acidic catalyst or alkali catalyst. Among the methods mentioned above, the aforementioned (1) is preferable in the present invention since it produces reduced amounts of impurities.

### [Oily component]

The oil composition for cosmetics material of the present invention comprises at least one oily component selected from silicone oil, ester oil, liquid oil, hydrocarbon, and fatty acid.

Examples of the silicone oil include methylpolysiloxane, methylphenylpolysiloxane, methylcyclopolysiloxane, decamethylcyclopentasiloxane, polyether-modified silicone, amino-modified silicone, betaine-modified silicone, alkyl-modified silicone, and alkoxy-modified silicone. These can be used each alone or in combination of two or more of them.

Examples of the ester oil include diisopropyl adipate, diisobutyl adipate, dioctyl adipate, di-2-hexyldecyl adipate, diisostearyl adipate, isostearyl myristate, isotridecyl myristate, isopropyl myristate, octyldodecyl myristate, cetyl octanoate, isononyl isononanoate, isodecyl isononanoate, isotridecyl isononanoate, diisopropyl sebacate, isopropyl palmitate, hexyl laurate, decyl oleate, hexyldecyl dimethyloctanoate, octyl palmitate, lauryl lactate, octyldodecyl lactate, isocetyl stearate, isocetyl isostearate, ethylene glycol dioctanoate, dipentaerythritol fatty acid ester, cetyl caprylate, glyceryl tricaprylate, neopentyl glycol dicaprate, and diisostearyl malate. These can be used each alone or in combination of two or more of them.

Other examples of oily components include liquid oil (triacylglycerol) that remains liquid at normal temperature (25°C) such as avocado oil, camellia oil, turtle oil, macadamia nut oil, corn oil, mink oil, olive oil, rapeseed oil, yolk oil, sesame oil, persic oil, wheat germ oil, sasanqua oil, castor oil, linseed oil, safflower oil, cotton seed oil, perilla oil, soybean oil, peanut oil, tea seed oil, torreya oil, rice bran oil, Chinese tung oil, Japanese tung oil, jojoba oil, germ oil, glyceryl trioctanoate, and glyceryl triisopalmitate; hydrocarbon such as liquid paraffin, squalene, squalane, and pristane; and fatty acid such as oleic acid, tall oil fatty acid, and isostearic acid. These can be used each alone or in combination of two or more of them.

### [Oil composition for cosmetics material]

The oil composition for cosmetics material of the present invention comprises 0.1 to 4.3% by weight of the glycerol fatty acid ester represented by the aforementioned formula (1), 0.5 to 25.7% by weight of the polyglycerol fatty acid ester represented by the aforementioned formula (2), 1 to 30% by weight of the polyglycerol monoalkyl ether represented by the aforementioned formula (3), 40 to 98.4% by weight of at least one oily component selected from silicone oil, ester oil, liquid oil, hydrocarbon, and fatty acid.

The glycerol fatty acid ester represented by the aforementioned formula (1) and the polyglycerol fatty acid ester represented by the aforementioned formula (2) have a liquid crystal formation-promoting function, while the polyglycerol monoalkyl ether represented by the aforementioned formula (3) has a liquid crystal formation-inhibiting function. Further, because the oil composition for cosmetics material of the present invention comprises the glycerol fatty acid ester represented by the aforementioned formula (1), the polyglycerol fatty acid ester represented by the aforementioned formula (2), and the polyglycerol monoalkyl ether represented by the aforementioned formula (3) at the aforementioned ratio, it can form fine emulsified particles via a stable lamellar liquid crystalline phase when mixed with water.

There is a tendency that when the amounts of the glycerol fatty acid ester represented by the aforementioned formula (1) and the polyglycerol fatty acid ester represented by the aforementioned formula (2) added exceed the aforementioned range, gel becomes too hard, resulting in reduced usability. Meanwhile, there is a tendency that when the amounts of the glycerol fatty acid ester represented by the aforementioned formula (1) and the polyglycerol fatty acid ester represented by the aforementioned formula (2) added are below the aforementioned range, formation of a lamellar liquid crystalline phase becomes difficult, resulting in reduced detergency and water solubility of the oil composition for cosmetics material.

It is preferable that the glycerol fatty acid ester represented by the aforementioned formula (1) and the polyglycerol fatty acid ester represented by the aforementioned formula (2) are incorporated in the oil composition for cosmetics material of the present invention at such a ratio that the HLB value is about 6 to 9, since the self-assembling capacity and lamellar liquid crystalline phase-forming property can be improved. Adjusting the blending ratio of the glycerol fatty acid ester represented by the aforementioned formula (1) to the polyglycerol fatty acid ester represented by the aforementioned formula (2) [former : latter (weight ratio)] to about 1.0 : 1.0 to 1.0 : 6.0 (especially, 1.0 : 1.0 to 1.0 : 5.0, preferably 1.0 : 1.0 to 1.0 : 4.5, and particularly preferably 1.0 : 1.1 to 1.0 : 4.5) is preferable from the viewpoints of cost and excellent lamellar liquid crystalline phase-forming property. There is a tendency that when the blending ratio of the glycerol fatty acid ester represented by the aforementioned formula (1) to the polyglycerol fatty acid ester represented by the aforementioned formula (2) is out of the aforementioned range, formation of a lamellar liquid crystalline phase becomes difficult and the detergency and water solubility of the oil composition for cosmetics material are reduced.

The amount of the polyglycerol monoalkyl ether represented by the aforementioned formula (3) added to the oil composition for cosmetics material is 1 to 30% by weight, and especially 1 to 20% by weight, particularly 1 to 15% by weight, is preferable from the viewpoints of cost and excellent lamellar liquid crystalline phase-forming property. There is a tendency that when the amount of the polyglycerol monoalkyl ether represented by the aforementioned formula (3) added exceeds the aforementioned range, gel becomes too hard, resulting in reduced usability. Meanwhile, there is a tendency that when the amount of the polyglycerol monoalkyl ether represented by the aforementioned formula (3) added is below the aforementioned range, formation of a lamellar liquid crystalline phase becomes difficult, resulting in reduced detergency and water solubility of the oil composition for cosmetics material.

Also, it is preferable to adjust the blending ratio of the glycerol fatty acid ester represented by the aforementioned formula (1) to the polyglycerol monoalkyl ether represented by the aforementioned formula (3) [former : latter (weight ratio)] in the oil composition for cosmetics material to, for example, about 1.0 : 0.3 to 1.0 : 135.0 (especially 1.0 : 0.5 to 1.0 : 32.0, preferably 1.0 : 0.8 to 1.0 : 16.0, particularly preferably 1.0 : 1.2 to 1.0 : 15.0, and most preferably 1.0 : 1 3.0 to 1.0 : 15.0) from the viewpoints of cost and excellent lamellar liquid crystalline phase-forming property.

Further, it is preferable to adjust the blending ratio of the polyglycerol fatty acid ester represented by the aforementioned formula (2) to the polyglycerol monoalkyl ether represented by the aforementioned formula (3) [former : latter (weight ratio)] in the oil composition for cosmetics material to, for example, about 1.0 : 0.3 to 1.0 : 22.0 (especially 1.0 : 0.5 to 1.0 : 4.6, preferably 1.0 : 0.8 to 1.0 : 4.0, particularly preferably 1.0 : 1.2 to 1.0 : 3.0) from the viewpoints of cost and excellent lamellar liquid crystalline phase-forming property.

There is a tendency that when the blending ratio of the glycerol fatty acid ester represented by the aforementioned formula (1), the polyglycerol fatty acid ester represented by the aforementioned formula (2), and the polyglycerol monoalkyl ether represented by the aforementioned formula (3) is out of the aforementioned range, formation of a lamellar liquid crystalline phase becomes difficult and that thereby the detergency and water solubility of the oil composition for cosmetics material are reduced.

The amount of the oily component added is 40 to 98.4% by weight, preferably about 40 to 75% by weight, particularly preferably 40 to 60% by weight, and most preferably 40 to 50% by weight of the total amount of the oil composition for cosmetics material. When the amount of the oily component added is below the aforementioned range, the amounts of the surfactants added are relatively increased, resulting in a tendency of increased irritation to the skin, etc.; meanwhile, when the amount of the oily component added exceeds the aforementioned range, the amounts of the surfactants added are relatively decreased, resulting in reduced solubility, making removal of the oily component from the skin surface difficult even by washing with water.

The oil composition for cosmetics material according to the present invention can be used as a cleansing agent for oily dirt and the like, a cleansing cosmetic for oily cosmetics and the like, a tanning oil, a baby oil, a hair oil, a foamy massage oil, and the like, and it can be particularly favorably used as a cleansing cosmetic for oily cosmetics and the like.

### [Cleansing cosmetic]

The cleansing cosmetic according to the present invention is characterized by comprising the aforementioned oil composition for cosmetics material. The content of the aforementioned oil composition for cosmetics material in the cleansing cosmetic can be appropriately adjusted by the amount of a liquid medium comprising aqueous components, and for example, the cleansing cosmetic may be a system rich in oily components (hereinbelow, may also be referred to as a "W/O system") or a system rich in aqueous components (hereinbelow, may also be referred to as an "O/W system"). It is preferable to use water as the liquid medium in the present invention.

Also, the cleansing cosmetic is not particularly limited to any of the lotion, solution, emulsion, cream, gel, and oil types; however, a cream type and a gel type are preferable from the viewpoint of excellent usability. These can be adjusted by the blending ratio of the oily component and aqueous component.

The content of the aforementioned oil composition for cosmetics material in a W/O system cleansing cosmetic is, for example, not less than 70% by weight, preferably about 70 to 95% by weight, and particularly preferably about 80 to 95% by weight. There is a tendency that when the content of the aforementioned oil composition for cosmetics material in the cleansing cosmetic is out of the aforementioned range, detergency is reduced.

The W/O system cleansing cosmetic of the present invention preferably has the following blending ratio.
Water: 3 to 50% by weight
Oily component: 35 to 67% by weight
Glycerol fatty acid ester: 1 to 5% by weight
Polyglycerol fatty acid ester: 2 to 8% by weight
Polyglycerol monoalkyl ether: 10 to 18% by weight

The content of the aforementioned oil composition for cosmetics material in an O/W system cleansing cosmetic is, for example, less than 70% by weight, preferably about 30 to 65% by weight, and particularly preferably about 35 to 60% by weight. There is a tendency that when the content of the aforementioned oil composition for cosmetics material in the cleansing cosmetic is out of the aforementioned range, detergency is reduced.

The O/W system cleansing cosmetic of the present invention preferably has the following blending ratio.
Water: 51 to 70% by weight
Oily component: 10 to 42% by weight
Glycerol fatty acid ester: 0.1 to 3.5% by weight
Polyglycerol fatty acid ester: 0.9 to 3.5% by weight
Polyglycerol monoalkyl ether: 5 to 13% by weight

Polyhydric alcohol may further be added to the cleansing cosmetic. Adding polyhydric alcohol can optimize the HLB value and also impart a moisturizing effect. Examples of the polyhydric alcohol include glycerol, diglycerol, maltitol, 1,3-butylene glycol, isoprene glycol, dipropylene glycol, polyethylene glycol, pentaerythritol, neopentyl glycol, sorbitol, sorbitan, trehalose, and propylene glycol. These can be used each alone or in combination of two or more of them.

In the present invention, among them, the cleansing cosmetic preferably comprises at least one selected from glycerol, maltitol, 1,3-butylene glycol, propylene glycol, and sorbitol. The content of polyhydric alcohol in the cleansing cosmetic is, for example, 5 to 70% by weight, preferably 10 to 50% by weight.

Further, additional components can be appropriately added to the cleansing cosmetic according to the present invention as needed within such a range that the objects of the present invention can be achieved. Examples of such an additional component include a nonionic surfactant other than those mentioned above, an anionic surfactant, an amphoteric surfactant, lower alcohols, a powder, an antioxidant, an antioxidant aid, an ultraviolet absorber, a humectant, an anti-inflammatory agent, an antiseptic agent, a pH adjuster, an extract derived from animals, plants, fishery products, and microorganisms, and a fragrance.

The nonionic surfactant other than those mentioned above is not particularly limited, and examples thereof include glycerol fatty acid ester other than those mentioned above, polyglycerol fatty acid ester other than those mentioned above, polyglycerol monoalkyl ether other than those mentioned above, polyalkylene glycol fatty acid ester, sorbitan fatty acid ester, sugar fatty acid ester, pentaerythritol fatty acid ester, polyoxyalkylene hydrogenated castor oil fatty acid ester, fatty acid alkanolamide, polyoxyalkylene glycol, ester of polyoxyalkylene glycol and monohydric or polyhydric alcohol, polyoxyalkylene sugar ether, a condensate of fatty acid amide and polyoxyalkylene glycol, a condensate of fatty acid amine and polyoxyalkylene glycol, and alkyl or alkenyl polyglycoside.

Examples of the anionic surfactant include, but are not particularly limited to, polyoxyethylene alkyl ether sulfate, alkyl sulfate salts, alkylbenzenesulfonic acid salts, α-olefin sulfonic acid salts, glutamic acid and other amino acid surfactants, N-acylmethyltaurine salts, and alkyl phosphate salts.

Examples of the amphoteric surfactant include, but are not particularly limited to, a carboxybetaine type, imidazolimium type, sulfobetaine type, and alanine type amphoteric surfactant.

Examples of the lower alcohol include, but are not particularly limited to, ethanol, propyl alcohol, ethylene glycol, and diethylene glycol.

Examples of the powder component include, but are not particularly limited to, an inorganic powder or an organic powder. Examples of the inorganic powder include talc, kaolin, mica, sericite, muscovite, phlogopite, synthetic mica, lepidolite, biotite, lithia mica, vermiculite, magnesium carbonate, zirconium silicate, aluminum silicate, barium silicate, calcium silicate, zinc silicate, magnesium silicate, strontium silicate, metal salts of tungstic acid, magnesium, silica, zeolite, barium sulfate, calcined calcium sulfate (calcined gypsum), calcium phosphate, fluoroapatite, hydroxyapatite, ceramic powder, activated carbon, medical carbon, metal soaps (such as zinc myristate, calcium palmitate, and aluminum stearate), and boron nitride. Examples of the organic powder include a polyamide resin powder (nylon powder), a polyethylene powder, a poly(methyl methacrylate) powder, a polystyrene powder, a styrene and acrylic acid copolymer resin powder, a benzoguanamine resin powder, and a cellulose powder.

Examples of the antioxidant include, but are not particularly limited to, vitamin E, dibutylhydroxytoluene, butylhydroxyanisole, and gallic acid esters.

Examples of the antioxidant aid include, but are not limited to, ascorbic acid, phytic acid, kephalin, and maleic acid.

Examples of the ultraviolet absorber include, but are not particularly limited to, a benzophenone derivative such as 2-hydroxy-4-methoxybenzophenone, 2-hydroxy-4-methoxybenzophenone-5-sulfonic acid, 2-hydroxy-4-methoxybenzophenone-5-sulfonic acid salt, and dihydroxydimethoxybenzophenone; p-aminobenzoic acid or its derivative such as p-aminobenzoic acid and ethyl p-aminobenzoate; a methoxycinnamic acid derivative such as ethyl p-methoxycinnamate, isopropyl p-methoxycinnamate, and octyl p-methoxycinnamate; a salicylic acid derivative such as octyl salicylate and phenyl salicylate; and urocanic acid, a urocanic acid derivative, 4-tert-butyl-4'-methoxydibenzoylmethane, 2-(hydroxy-5'-methylphenyl)benzotriazole, and methyl anthranilate.

Examples of the humectant include sodium lactate, pyrrolidone carboxylic acid, and a pyrrolidone carboxylic acid derivative.

Examples of the anti-inflammatory agent include, but are not particularly limited to, glycyrrhizic acid, a glycyrrhizic acid derivative, glycyrrhetic acid, a glycyrrhetic acid derivative, allantoin, hydrocortisone acetate, and azulene.

Examples of the antiseptic agent include, but are not particularly limited to, methyl paraben, propyl paraben, and phenoxy ethanol.

Examples of the pH adjuster include, but are not particularly limited to, citric acid, hydrochloric acid, sulfuric acid, phosphoric acid, sodium hydroxide, and ammonia.

Examples of the extract derived from animals, plants, fishery products, and microorganisms include, but are not particularly limited to, an extract such as a tea extract, an aloe extract, a ginkgo extract, a Swertia herb extract, a mugwort extract, a garlic extract, a Scutellaria root extract, a rosemary extract, a Luffa extract, a placental extract, an extract from lactic acid bacteria culture, and a seaweed extract.

The fragrance is not particularly limited as long as it is one that is commonly used in cosmetics.

The cleansing cosmetic according to the present invention comprises the aforementioned oil composition for cosmetics material, and therefore, either on wet or dry skin, it can be readily miscible with makeup dirt on the skin surface and then freshly rinsed off by washing with water without leaving an oily feel, and be favorably used even in a bath. Moreover, the cleansing cosmetic of the present invention has excellent usability, safety, and stability over time.

### Examples

Hereinbelow, the present invention will be more specifically described with reference to Examples; however, the present invention is not limited to these Examples.

### Examples 1 to 6, Comparative Examples 1 to 6

Cleansing cosmetics having the blending formulations (unit: weight part) shown in Table 1 below were prepared according to a customary procedure, and the detergency, solubility, and lamellar liquid crystalline phase-forming property were evaluated based on the following criteria.

### (Evaluation method and evaluation criteria)

### (1) Detergency (under dry conditions)

A Lipstick (trade name, "Sofina AUBE couture designing stay rouge RD532," available from Kao Corporation) was applied to the forearm, and the cleansing cosmetics obtained in Examples and Comparative Examples were each scooped by hand, about 0.5 g each, with which the forearm was massaged 20 times, followed by washing with water. Subsequently, the amount of lipstick washed off was visually observed and evaluated based on the following criteria.
<Evaluation criteria>
Excellent: Completely washed off
Good: Almost completely washed off
Fair: Still slightly remained
Poor: Almost entirely remained

### (2) Detergency (under wet conditions)

A Lipstick (trade name, "Sofina AUBE couture designing stay rouge RD532," available from Kao Corporation) was applied to the forearm, then the forearm got wet with water, and the cleansing cosmetics obtained in Examples and Comparative Examples were each scooped by hand, about 0.5 g each, with which the forearm was massaged 20 times, followed by washing with water. Subsequently, the amount of lipstick washed off was visually observed and evaluated based on the above criteria.

### (3) Solubility

To 0.5 g of each of the cleansing cosmetics obtained in Examples and Comparative Examples, 0.5 g of water was added, followed by mixing thoroughly, and then the resulting mixture was visually observed to confirm whether it was transparent or not and evaluated based on the following criteria.
<Evaluation criteria>
Good: Transparent
Poor: Opaque

### (4) Lamellar liquid crystalline phase-forming property

To 0.5 g of each of the cleansing cosmetics obtained in Examples and Comparative Examples, 0.5 g of water was added, followed by mixing thoroughly, and then the resulting mixture was observed with a polarizing plate to confirm whether it formed a lamellar liquid crystalline phase or not and evaluated based on the following criteria.
<Evaluation criteria>
Good: Polarized
Poor: Not polarized

[Table 1]

**Table 1**

| | | | | Example | | | | | | Comparative Example | | | | | |
|---|---|---|---|---|---|---|---|---|---|---|---|---|---|---|---|
| | | | | 1 | 2 | 3 | 4 | 5 | 6 | 1 | 2 | 3 | 4 | 5 | 6 |
| Water | | Distilled water | | 37.50 | 37.50 | 37.50 | 37.50 | 37.50 | 37.50 | 37.50 | 37.50 | 37.50 | 37.50 | 37.50 | 37.50 |
| Oily component | | Cetyl octanoate | | 43.75 | 29.17 | 29.17 | 43.75 | 43.75 | 43.75 | 43.75 | | 43.75 | 43.75 | 43.75 | 43.75 |
| | | Liquid paraffin | | | 14.58 | | | | | | | | | | |
| | | Decamethylpentasiloxane | | | | 14.58 | | | | | 43.75 | | | | |
| Surfactant | (1) | Monoglycerol monooleate | | 2.11 | 2.11 | 2.11 | 2.11 | 4.68 | 1.31 | 18.75 | | 4.69 | 2.11 | | 2.11 |
| | (2) | Diglycerol monooleate | | 6.33 | 6.33 | 6.33 | 6.33 | 4.68 | 5.25 | | 18.75 | 14.06 | | 4.68 | 6.33 |
| | | Diglycerol isostearate | | | | | | | | | | | 6.33 | 4.68 | |
| | | Decaglycerol isostearate | | | | | | | | | | | 10.31 | | 10.31 |
| | (3) | Heptaglycerol monoisostearyl ether | | | | | | 9.39 | | | | | | 9.39 | |
| | | Hexaglycerol monoisostearyl ether | | | | | | | 12.19 | | | | | | |
| | | Pentaglycerol monoisostearyl ether | | 10.31 | 10.31 | 10.31 | | | | | | | | | |
| | | Hexaglycerol mono 2-hexyldecyl ether | | | | | 10.31 | | | | | | | | |
| Evaluation | | Detergency | Under dry conditions | Excellent | Excellent | Excellent | Excellent | Excellent | Excellent | Poor | Poor | Poor | Poor | Poor | Poor |
| | | | Under wet conditions | Excellent | Excellent | Excellent | Excellent | Excellent | Excellent | Poor | Poor | Poor | Poor | Poor | Poor |
| | | Solubility | | Good | Good | Good | Good | Good | Good | Poor | Poor | Poor | Poor | Poor | Poor |
| | | Lamellar liquid crystalline phase-forming property | | Good | Good | Good | Good | Good | Good | Poor | Poor | Poor | Poor | Poor | Poor |

As apparent from Table above, the cleansing cosmetics comprising the oil composition for cosmetics material according to the present invention form a lamellar liquid crystalline phase and are readily miscible with makeup dirt, even on wet or dry skin, thereby exerting excellent cleansing ability. Moreover, the cleansing cosmetics comprising the oil composition for cosmetics material according to the present invention have excellent water solubility, and therefore, can be rinsed off by washing with water rapidly without leaving an oily feel, thereby exerting excellent usability.

### Industrial Applicability

The oil composition for cosmetics material according to the present invention has excellent detergency and usability, and can be used even in a bath. Also, a cleansing cosmetic of desired type such as a cream, emulsion, oil, or oily gel type cleansing cosmetic can be produced by adjusting the content of the oily component. Thus, the cleansing cosmetic according to the present invention is particularly useful as a cleansing agent for oily cosmetics.

## Claims

1. An oil composition for cosmetics material, comprising
0.1 to 4.3% by weight of glycerol fatty acid ester represented by the following formula (1) :
R₁COO-C₃H₆O₂-H (1)
wherein R₁ represents a linear or branched aliphatic hydrocarbon group having 8 to 22 carbon atoms that optionally has a hydroxyl group,
0.5 to 25.7% by weight of polyglycerol fatty acid ester represented by the following formulae (2) :
R₂COO-(C₃H₆O₂)n₁-H (2)
wherein R₂ represents a linear or branched aliphatic hydrocarbon group having 8 to 22 carbon atoms that optionally has a hydroxyl group, and n₁ represents an average degree of polymerization of glycerol, which is 2 to 10,
1 to 30% by weight of Polyglycerol monoalkyl ether represented by the following formula (3) :
R_{3O}- (C₃H₆O₂) n₂-H (3)
wherein R₃ represents a linear or branched alkyl group having 8 to 22 carbon atoms that optionally has a hydroxyl group, and n₂ represents an average degree of polymerization of glycerol, which is 3 to 20, and
40 to 98.4% by weight of at least one oily component selected from silicone oil, ester oil, liquid oil, hydrocarbon, and fatty acid.

2. A cleansing cosmetic, comprising the oil composition for cosmetics material according to claim 1.
